# EUROPEAN PATENT APPLICATION

(11) **EP 4 481 756 A1**
(43) Date of publication of application: **25.12.2024**
(21) Application number: 23180068.1
(22) Date of filing: 19.06.2023
(51) Int. Cl.: G16H 20/70, G16H 40/63, G16H 50/30, G16H 50/70, A61B 5/00, G06N 20/00, G06V 40/00

(54) **CLINICAL PREPARATORY ENVIRONMENT**

(71) Applicant: Koninklijke Philips N.V., 5656 AG Eindhoven (NL)
(72) Inventor: KUHLMANN, Daan, Eindhoven (NL); BUIL, Vincentius Paulus, Eindhoven (NL); SPELT, Hanne Adriana Alijda, 5656AG Eindhoven (NL); VAN EE, Raymond, Eindhoven (NL); NAUTS, Sanne, Eindhoven (NL); KLAASSEN, Remy, Eindhoven (NL); BULUT, Murtaza, Eindhoven (NL); BEREZHNOY, Igor, Eindhoven (NL)
(74) Representative: Philips Intellectual Property & Standards

(57) **Abstract**

Proposed concepts thus aim to provide schemes, solutions, concept, designs, methods and systems pertaining to controlling a clinical preparatory environment. In particular, embodiments aim to provide a method for controlling a clinical preparatory environment. This can be achieved by determining a psychological state of a subject in a clinical preparatory environment, as well as a system state of a system in the same environment. Based on this data, an adjustment for the system can be determined that might, for example, change the psychological state of the subject in a positive manner. After determining the adjustment based on the psychological state and a system state, a control signal can then be generated for controlling a human-perceivable output of the system, such that the subject may notice this output and be affected in some way.

## Description

### FIELD OF THE INVENTION

This invention relates to the field of controlling a clinical preparatory environment, and more specifically wherein the environment comprises a system.

### BACKGROUND OF THE INVENTION

For patients, undergoing a diagnostic examination (e.g., a CT, MRI or X-ray) or hospital procedure (e.g., radiotherapy, procedure, imaging guided therapy) can be a stressful experience. This is particularly true for children and other vulnerable patient groups, such as elderly patients and patients with a developmental delay. If patients/subjects are highly anxious, this can negatively affect not only their experience but also the hospital workflow or the quality of diagnostic images/therapy outcomes.

Preparing subjects before a scan or procedure can help improve Patient and Staff Experience, as well as a subject's ability and willingness to comply with instructions, e.g., the instruction to lie still during a scan.

Clinical preparatory environments, such as waiting rooms or playrooms, are often used in hospitals to prepare subjects for certain exams or therapies. These environments may often include preparatory systems, such as miniature versions of a CT and/or an MRI machine with sensors and a connected display. These can be used to prepare children and/or adults for an imaging exam. Other preparatory systems may be present in the environment such as mock-up scanner, preparatory toys, preparatory movies, preparatory books, brochures, coloring books, interactive games, or movies, etc. These systems may be used by subjects by themselves and without any guidance from a healthcare provider. Subjects who are particularly anxious or withdrawn may not initiate interactions with preparatory systems, however, even though they are likely to be those who would benefit most from good preparation.

### SUMMARY OF THE INVENTION

The invention is defined by the claims.

According to examples in accordance with an aspect of the invention, there is provided a method for controlling a clinical preparatory environment, wherein the clinical preparatory environment comprises a system configured to output a human-perceivable output.

The method comprises: determining a psychological state of a subject in the clinical preparatory environment; determining a system state describing an operating state of the system; determining an adjustment for the system based on the psychological state of the subject and the system state; and generating, based on the determined adjustment, a control signal for controlling the human-perceivable output of the system.

Proposed concepts thus aim to provide schemes, solutions, concepts, designs, methods and systems pertaining to controlling a clinical preparatory environment. In particular, embodiments aim to provide a method for controlling a clinical preparatory environment comprising a system by determining a psychological state of a subject as well as a system state. These states can then be used to generate a control signal for controlling the system based on a determined adjustment for the system.

In other words, it is proposed that by determining a psychological state of a subject in a clinical preparatory environment, an adjustment for a system also in the environment can be determined that might, for example, change the psychological state of the subject in a positive manner. After determining the adjustment based on the psychological state and a system state, a control signal can then be generated for controlling a human-perceivable output of the system, such that the subject may notice this output and be affected in some way.

By considering the psychological state of a subject as well as the current state of a system in the same environment, an adjustment for the system can be determined that may be noticed by the subject and may induce a change in their psychological state. In a simple example, for instance, the system in the environment may be a preparatory system configured for preparing a subject for a clinical procedure via interaction with the subject, and on determining that the system is idle and that the subject is anxious and/or has their attention focused on something else, an adjustment of activating the system may be determined.

In another example, the system may be a sound system in the environment, and on determining that the subject is stressed, an adjustment of controlling the sound system to play calming music may be determined. In yet another example, the system may be a display system in the environment, and on determining that the subject is bored, an adjustment of controlling the display to start playing a video may be determined.

Essentially, the present invention allows for a system in a clinical preparatory environment to react dynamically and automatically to the moods, emotions, and behaviour of a subject in the environment in order to try and affect the subject's psychological state positively. In cases in which the subject is a child and the system is a preparatory system designed to prepare children for a procedure, the invention facilitates the automatic and dynamic guidance of the child towards interacting with the preparatory system.

Further, as the time of health care providers is scarce, the invention is of particular benefit in busy hospitals or clinical facilities, as it provides a method capable of continuously and unobtrusively observing behaviour and controlling the environment to positively affect the psychological state of a subject awaiting a procedure.

Ultimately, an improved method for controlling a clinical preparatory environment is provided by the present invention.

In some embodiments, the system may be a preparatory system configured for preparing a subject for a clinical procedure via interaction with the subject. Preparatory systems provide effective ways of preparing subjects for clinical procedures, especially children. Preparatory systems may simulate the clinical procedure and/or walk the subject through the steps that will be taken, explaining how and why they are being performed. Preparatory systems may also provide advice for the clinical procedure. Simply by interacting with a preparatory system before the real procedure and becoming more familiar with it, subjects' anxieties may be reduced both before the procedure and during it. An example of preparatory system is a mini mock-CT or MRI scanner.

In some embodiments, the human-perceivable output of the system may comprise at least one of: lighting; sound; projections; content on a display; music; vocals; movement; touch; temperature; fragrance; speech; look; feel; and interaction style. These are system outputs that can be adjusted and readily noticed and/or appreciated by subjects, such that their psychological state may be changed. For instance, the lights of the system may change colour or intensity; the system may emit a sound; the system may project an image or video on a surface; the system may play a video on a display; the system may play music; the system may speak or sing; the system may move a part of itself or the whole system may move; the system may touch a subject; the system may change the temperature of itself or the environment; the system may emit a fragrance; the system may talk with the subject; the system may change how it looks; and the system may change how it feels when touched, for instance, turning harder or softer. Interaction style can be defined in terms how different human-perceivable outputs of the system are combined and generated, for instance, in which order, at what intensity, in what direction, at what time, etc. For example, in the case of speech-based interaction, the interaction style can include semantic and lexical aspects such as sentence structure, words used, dialogue structure, length of sentences, length of silences, etc.

In some embodiments, the system may be an environmental system configured for controlling at least one environmental parameter of the preparatory environment. For instance, the environmental parameter may be a temperature of the environment; music playing in the environment; the lighting of the environment; content playing on displays in the environment; and/or videos or games being projected in the environment. All these parameters can be adjusted to affect the psychological state of the subject, for instance, to put them more at ease.

In some embodiments, determining a psychological state of a subject may comprise: obtaining subject information describing at least one of a physiological and a psychological parameter of the subject; and determining the psychological state of the subject based on the obtained subject information. By obtaining information describing a physiological and/or psychological parameter of a subject, the psychological state of the subject may be determined based on this information. For instance, subject information can be obtained describing that the subject has been sitting still for twenty minutes or that the subject is fidgeting; that the subject has elevated heart rate; that the subject is clinging to their parents; or that the subject is simply looking away from the system. From these psychological/physiological parameters, a psychological state of the subject can be determined, such as the subject being stressed or the subject not having their attention focused on the system. The subject information can be provided by a user, for example, by staff or parents.

In some embodiments, obtaining subject information may comprise obtaining sensor data of the subject describing at least one of a physiological and a psychological parameter of the subject. By obtaining sensor data, a physiological and/or a psychological parameter of the subject can be determined directly. For instance, the sensor may be a camera and/or microphone in the environment, a ceiling-camera, and/or a heart rate monitor worn by the subject. From this sensor data, the psychological state of the subject can be determined. The use of sensor data thus provides an information source that can be provided automatically, unobtrusively, and continuously whilst the subject is in the waiting area and within the sensors' purview.

In some embodiments, the sensor data may comprise at least one of: optical data; sound data; speech data; image data; movement data; accelerometer data; gyroscope data; haptic data; sweat data; electrodermal activity data; cardiovascular activity data; respiratory activity data; electroencephalography (EEG) data; temperature data; and touch data. All this data can be beneficial in determining the psychological state of a subject. Optical data can be acquired via a camera, for example. Sound or speech data can be acquired via a microphone, for example. Cardiovascular activity data can include different variability parameters such as SDNN, RMSSD, high/low frequencies, heart rate, and/or blood pressure, etc. Electrodermal activity data can include galvanic skin response and/or galvanic skin level.

In some embodiments, a psychological state of the subject may comprise at least one of: an attention direction of the subject; an anxiety value of the subject; a stress value of the subject; a verbal value of the subject; and a predominant emotion of the subject. All these psychological states are states beneficial to monitor and perhaps change via a system adjustment so that the subject can be better prepared for the clinical procedure. It should be noted that both stress and anxiety are emotions, and that stress is a temporary response to a certain trigger whereas anxiety is prolonged, can linger, and may not have a specific trigger. A verbal value of the subject can be understood as how much, how loudly, and/or to whom the subject is talking.

In some embodiments, determining an adjustment for the subject may comprise processing the psychological state of the subject and the system state with an adjustment algorithm to determine an adjustment for the system. In this way, both the psychological state of the subject and the system state can be considered together, thus more efficiently and/or effectively determining an adjustment for the system. For example, the adjustment algorithm may be a rule-based algorithm.

In some embodiments, the adjustment algorithm may be a machine-learning algorithm trained to predict, based on the psychological state of the subject and the system state, an adjustment for the system. Machine-learning algorithms can provide a more efficient and/or effective way to process information and provide outputs. For instance, the machine-learning algorithm may be a convolutional neural network or a random forest classifier, both beneficial for processing multiple inputs.

In some embodiments, after generating the control signal, the method may further comprise: determining a further psychological state of the subject; and adjusting the adjustment algorithm based on the further psychological state. In this way, the algorithm can learn from its previous determined adjustments, for example, to learn which adjustments positively affected the psychological state of the subject, did not affect the state at all, or, in fact, negatively affected it.

In some embodiments, the method may further comprise: determining an identity of the subject; obtaining a profile of the subject based on the identity of the subject, wherein the profile describes at least one of: at least one trait of the subject; and at least one previous interaction of the subject with the system; and at least one of: determining the psychological state of the subject based on the profile of the subject; and determining an adjustment for the system further based on the profile of the subject. The identity of the subject can be used to obtain a profile of the subject describing their traits, such as whether they are naturally anxious, their age, demographics, and/or their previous interactions with the system. This information can then be used to more accurately determine the subject's psychological state and/or determine a more effective system adjustment for affecting their psychological state. For example, if the subject's profile describes that they are naturally anxious, then this information is useful in determining their psychological state. Further, if the subject's profile describes that they previously interacted with the system only after a certain adjustment was made, then the same adjustment can be determined again.

In some embodiments, a physiological parameter may comprise at least one motion parameter. A motion parameter can be understood as a parameter relating to the motion of the subject, for example, how the subject moves, their posture, what body parts of the subject are moving, the speed of the subject's movement or their body parts, the certainty of their movement, and/or the direction of their movement.

According to another aspect of the invention, there is provided a computer program comprising code means for implementing the method of any herein disclosed method when said program is run on a processing system.

According to another aspect of the invention, there is provided a controlling apparatus for controlling a clinical preparatory environment, wherein the clinical preparatory environment comprises a system configured to output a human-perceivable output. The controlling apparatus comprises: a processing unit configured to: determine a psychological state of a subject in the clinical preparatory environment; determine a system state describing an operating state of the system; and determine an adjustment for the system based on the psychological state of the subject and the system state; and an output interface configured to:
generate, based on the determined adjustment, a control signal for controlling the human-perceivable output of the system.

In some embodiments, the system may be a preparatory system configured for preparing a subject for a clinical procedure via interaction with the subject.

Thus, there may be proposed concepts for controlling a clinical preparatory environment, and this may be done based on the determining and controlling of an adjustment to be made to the outputs of a system in the environment, based on the psychological state of a subject and a system state of the system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for controlling a clinical preparatory environment according to a proposed embodiment;
Fig. 2 is a flow diagram of a method for controlling a clinical preparatory environment according to a proposed embodiment;
Fig. 3 is a more in-depth flow diagram of a method for controlling a clinical preparatory environment according to a proposed embodiment;
Fig. 4 is a simplified block diagram of a controlling apparatus for controlling a clinical preparatory environment according to a proposed embodiment;
Fig. 5 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to controlling a clinical preparatory environment. According to proposed concepts, a number of possible solutions may be implemented separately or jointly. That is, although these possible solutions may be described below separately, two or more of these possible solutions may be implemented in one combination or another.

Embodiments of the invention aim to provide a method for controlling a clinical preparatory environment. This can be achieved by determining a psychological state of a subject in a clinical preparatory environment, as well as a system state of a system in the same environment. Based on this data, an adjustment for the system can be determined that might, for example, change the psychological state of the subject in a positive manner. After determining the adjustment based on the psychological state and a system state, a control signal can then be generated for controlling a human-perceivable output of the system, such that the subject may notice this output and be affected in some way.

By considering the psychological state of a subject as well as the current state of a system in the same environment, an adjustment for the system can be determined that may be noticed by the subject and may induce a change in their psychological state. In a simple example, for instance, the system in the environment may be a preparatory system configured for preparing a subject for a clinical procedure via interaction with the subject, and on determining that the system is idle and that the subject is anxious and/or has their attention focused on something else, an adjustment of activating the system may be determined.

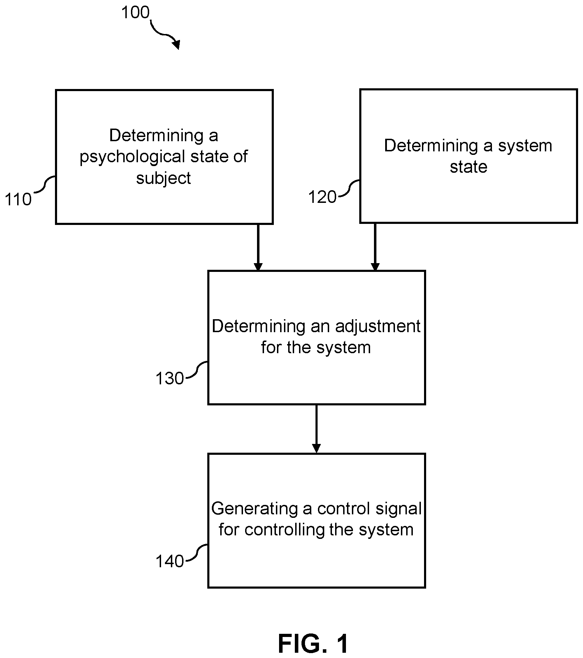

Referring now to Fig. 1, there is depicted a simplified flow diagram of a method 100 for controlling a clinical preparatory environment according to a proposed embodiment, wherein the clinical preparatory environment comprises a system configured to output a human-perceivable output.

The method 100 begins with the step 110 of determining a psychological state of a subject in the clinical preparatory environment. The clinical preparatory environment may be, for example, a waiting room, playroom, playground, or any other room the subject resides in before a clinical procedure or exam in a hospital or another medical facility. The clinical preparatory environment may alternatively be a virtual environment which the subject can access via a virtual reality headset. In some embodiments, the clinical preparatory environment may be an augmented reality environment, i.e., a mixture of physical and virtual systems which the subject can interact with via an augmented reality headset. In these cases, the system may be a virtual system configured to output a human-perceivable output.

The psychological state of the subject may also be understood as a behavioural state of the subject. States are characteristics patterns in a specific situation at a specific temporary time.

In this embodiment, the psychological state of the subject comprises an anxiety value of the subject. In various embodiments, the anxiety value may be a value between 0-100; a percentage value; a label, such as, for example, mildly anxious; or any other suitable system for providing a value indicating a level of anxiety of the subject.

In other embodiments, a psychological state of the subject may comprise, in addition to or as an alternative to an anxiety value, at least one of: an attention direction of the subject; a stress value of the subject; a verbal value of the subject; and a predominant emotion of the subject. All these psychological states are states beneficial to monitor and perhaps change via a system adjustment so that the subject can be better prepared for the clinical procedure. It should be noted that both stress and anxiety are emotions, and that stress is a temporary response to a certain trigger whereas anxiety is prolonged, can linger, and may not have a specific trigger. A verbal value of the subject can be understood as how much, how loudly, and/or to whom the subject is talking. A predominant emotion can be understood as a prediction of what emotion the subject is currently feeling most, e.g., stress, excitement, sadness, boredom, etc.

An attention direction of the subject may comprise a gaze direction of the subject and/or an interaction target of the subject. An interaction target of the subject may be, for example, another subject, parent, caregiver, toy, display, book, or device, etc. How long the subject interacts with an interaction target may also be determined and can be included in their psychological state.

Behaviour (psychological states) can be analysed in different ways, for example, by creating an algorithm that is based on an observational scale (such as the mYPAS) or using known algorithms for modelling user emotions on physiology (Mandryk, 2008).

In step 120, a system state is determined describing an operating state of the system. An operating state of the system can be understood as how the system is currently operating, and/or what the system's current human-perceivable outputs are. For example, the operating state of the system can comprise whether the system is currently active or idle, what colour light it is emitting, what sounds it is emitting, what data is communicating to other systems, etc.

In this embodiment, the system is a preparatory system configured for preparing a subject for a clinical procedure via interaction with the subject. Preparatory systems provide effective ways of preparing a subject for a clinical procedure, especially children. Preparatory systems may simulate the clinical procedure and/or walk the subject through the steps that will be taken, explaining how and why they are being performed. Preparatory systems may also provide advice for the clinical procedure. Simply by interacting with a preparatory system before the real procedure and becoming more familiar with it, subjects' anxieties may be reduced both before the procedure and during it.

For example, the preparatory system may be a mini-CT or mini-MRI scanner, and may have human-perceivable outputs comprising lights, sounds, speech, content on displays, etc. Its operating state could include whether the display is on, whether the lights are on and what colour they are, whether another subject is currently interacting with it, what sounds it is emitting, etc. The preparatory system is configured to reassure and prepare subjects (typically children), through interaction with them, for a real clinical procedure.

However, subjects may not interact with the preparatory system, or even if they do, the output of the system may not sufficiently reassure the subject. Therefore, an adjustment can be determined to provoke the subject into interacting with the system and/or more effectively affecting their psychological state during interaction.

A human-perceivable output can be understood as an output of a system detectable by a typical human, person, subject and/or user, in the sense that it is discernible by typical human sight, hearing, touch, smell, or taste.

It should be noted that the preparatory system may also be used to help prepare a subject not only for a procedure but for discharge after a procedure. This can help relax a subject after a clinical event, as well as increase their familiarity/relation with the system, which can help with follow-up visits.

In some embodiments, the human-perceivable output of the system may comprise at least one of: lighting; sound; projections; content on a display; music; vocals; movement; touch; temperature; fragrance; speech; look; and feel. These are system outputs that can be adjusted and readily noticed and/or appreciated by subjects, such that their psychological state may be changed. For instance, the lights of the system may change colour or intensity; the system may emit a sound; the system may project an image or video on a surface; the system may play a video on a display; the system may play music; the system may speak or sing; the system may move a part of itself or the whole system may move; the system may touch a subject; the system may change the temperature of itself or the environment; the system may emit a fragrance; the system may talk with the subject; the system may change how it looks; and the system may change how it feels when touched, for instance, turning harder or softer.

In other embodiments, instead of a preparatory system, the system may be an environmental system configured for controlling at least one environmental parameter of the preparatory environment. For instance, the environmental parameter may be a temperature of the environment, music playing in the environment, the lighting of the environment, content playing on displays in the environment, and/or videos or games being projected in the environment. All these parameters can be adjusted to affect the psychological state of the subject, for instance, to put them more at ease. An environmental system can be understood as any system which might affect an environmental parameter of the preparatory environment. For example, in some embodiments, the environmental system may comprise at least one of: an air conditioning system; a sound system in the environment; displays in the environment; lights in the environment; fragrance-emitters in the environment; and projectors in the environment.

In step 130, an adjustment for the system is determined based on the psychological state of the subject and the system state. For example, if it has been determined that the subject has a high anxiety value, and that the preparatory system is currently not being interacted with, an adjustment may be determined to have the preparatory system invite the subject over to interact with it via speaking through its speakers. For instance, a speech audio file may be played through the preparatory system's speakers, or alternatively speech may be dynamically generated using AI vocals or traditional text-to-speech techniques.

The determined adjustment can be a direct adjustment to the human-perceivable outputs of the system, e.g. immediately changing colour of the lights of the system, or it may be an indirect adjustment, e.g., adjusting how the system will react to interactions, such as, changing what sound will play when a certain button is pressed.

Further, if while the subject is interacting with the system, their psychological state is not improving, or is becoming worse, the determined adjustment can be for more effectively trying to improve the psychological state of the subject. In essence, determined adjustments for preparatory systems thus largely fall in two categories: either enticing the subject to interact with the system; or affecting their psychological state while they are interacting the subject.

Determined adjustments for environmental systems also largely fall in two categories: inviting the subject to interact or play with a preparatory system, e.g. via subject-targeted projections or displayed content; or making the environment more comfortable for the subject, e.g. by adjusting environmental lighting, sounds, etc. In some embodiments, an adjustment may be to inform a subject's caregiver via projections or displayed content about ways to comfort the subject, or simply instructing the caregiver to comfort them. In some embodiments, an adjustment may also comprise informing health care professionals or a caregiver of a subject's psychological state via content displayed on a device or any other suitable method such as information displayed on a dashboard. For example, the adjustment can be to invite the caregiver to start engaging with the subject or them to interact with a preparatory system themselves in order to perhaps prompt the subject to join in. If the subject does start interacting with the preparatory system, a further adjustment may then be determined to inform the caregiver to leave and let the subject continue alone.

In other words, based on behavioural analysis, the behaviour of a system (for instance, a preparatory or environmental system) can be adapted in order to try and improve the subject's behaviour. For example, children may be stimulated to interact with a preparatory system by determining an adjustment of the system (or another environmental system in the same preparatory environment) which involves actuating technology, such as floor projections, robotics, or any other suitable means. Further, upon detection of anxious and/or unmotivated behaviour, an adjustment to begin a presentation of multisensory rhythmically synchronous stimulation (audio and video with features that change rhythmically). Such stimulations are known to distract subjects from anxiety and to motivate the subject by focusing their attention.

In other words, based on the subject's calculated anxiety level, the system generates appropriate interventions to reassure the subject, via, for example, a library of effective anxiety-intervention mappings. These interventions may concern adaptive preparatory system and/or environmental behaviour. For example, a (virtual) character could be sent to a subject via displays in the environment, (floor) projections, robotics, or other means, inviting them to play with the preparatory system. Alternatively, the behaviour of the preparatory system may be directly adjusted. In some embodiments, these interventions may also concern adapting workflow of hospital staff, such as sending in specialized child life experts to comfort these children in the waiting room, and/or informing scanning staff that they may need more time with these children during scanning.

In some embodiments, the adjustment may be configured to invite subjects to interact with each other using projections and/or displayed content. This can include 'matching' subjects based on their anxiety level. For example, a subject with a low level of anxiety may be stimulated to interact with a more anxious subject. Interacting with another anxious subject may not be very helpful for highly anxious children, while interacting with a subject who is less anxious can be beneficial.

In step 140, a control signal for controlling the human-perceivable output of the system is generated based on the determined adjustment. By generating a control signal, embodiments may support interacting with a wide variety of systems, so that the current invention is not limited to controlling only a specific system or set of systems.

In embodiments in which multiple subjects are present in the same environment, and different subjects have different needs with regards to adjustments that may be incompatible, the method can take different factors into account. For example, the adjustments can be prioritized according to the timings of the clinical procedures, e.g., which subject's procedure will take place first; subjects' relative anxiety levels; subject' relative stress levels; or a combination thereof. As an alternative to prioritizing one subject over another, an average anxiety/stress level across subjects can be used as input to select an adjustment.

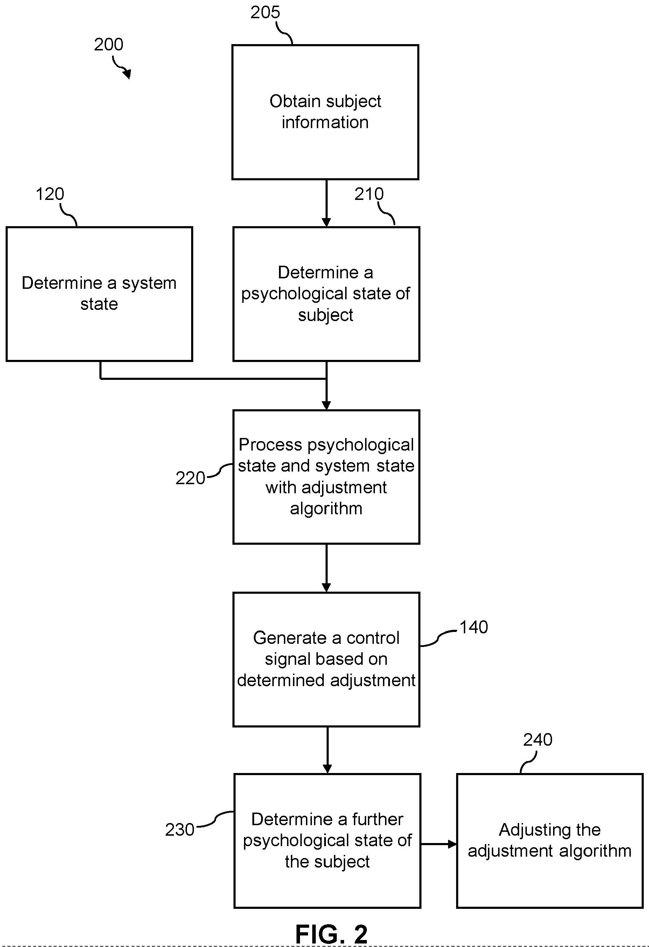

Referring now to Fig. 2, there is depicted a flow diagram of a method 200 for controlling a clinical preparatory environment according to a proposed embodiment, wherein the clinical preparatory environment comprises a system configured to output a human-perceivable output.

Steps 120 and 140 are substantially the same as have been described in relation to the method 100 in Fig. 1.

In step 205, subject information is obtained describing at least one of a physiological and a psychological parameter of the subject. By obtaining information describing a physiological and/or psychological parameter of a subject, the psychological state of the subject can be determined based on this information. For instance, subject information can be obtained describing that the subject has been sat still for twenty minutes or that the subject is fidgeting; that the subject has elevated heart rate; that the subject is clinging to their parents; and/or that the subject is simply looking away from the system. From these physiological/psychological parameters, a psychological state of the subject can be determined, such as the subject being stressed or the subject not having their attention focused on the system. Subject information may be obtained via sensors or alternatively, or in addition, it may be manually input by a user, for example, observing the subject. Subject information may also be understood as behavioural information describing at least one behavioural parameter. A behavioural parameter can comprise outward behaviour or inner behaviour, such as heart rate, breathing rate, etc. If input by a user, for example a clinician or a parent or self-reported, the subject information may be obtained using questionnaires, for example.

In step 210, a psychological state of the subject is determined based on the subject information obtained in step 205. In some embodiments, step 210 may comprise providing the subject information as input to a machine-learning algorithm trained to predict, for the subject information, a psychological state of the subject.

In other words, in step 210, while a subject is in the clinical preparatory environment, their physiology, facial expression and/or behaviour can be measured. Behaviour can be with respect to at least one of: a caregiver; other subjects; a preparatory device; toys; and a doll. The behaviour can be assessed with an observational scale. This can include measurements of gaze, e.g., if the subject is looking down or at a parent, etc.; motion, e.g., whether the subject is sitting still or exploring or shaking, etc.; vocalisations, e.g., whether the subject is quiet, engaging in `baby talk', or crying, etc.; and facial expressions. In some embodiments, this information is provided to a user to make a decision and input the subject's psychological state, and in other embodiments, this is automatically done via algorithms and/or machine-learning algorithms.

In step 220, the psychological state of the subject and the system state are processed with an adjustment algorithm to determine an adjustment for the system. In this way, both the psychological state of the subject and the system state can be considered together, thus more efficiently and/or effectively determining an adjustment for the system. For example, the adjustment algorithm may comprise a decision tree, a look-up table, and/or behaviour-intervention mapping.

In step 230, after generating the control signal in step 140, a further psychological state of the subject is determined. This may be determined by obtaining further subject information after generating the control signal in a step (not shown) much like step 205. In step 240, the adjustment algorithm is adjusted based on the further psychological state. In this way, the algorithm can learn from its previous determined adjustments, for example, to learn which adjustments positively affected the psychological state of the subject, didn't affect the state at all, or, in fact, negatively affected it. For example, in embodiments in which the adjustment algorithm comprises a decision tree, the tree may be adjusted to reflect the success or failure of a determined adjustment to change the psychological state of the subject. For example, if the further psychological state of the subject shows that a determined adjustment successfully calmed a stressed subject, then the adjustment algorithm can be adjusted to more frequently determine the same adjustment for other stressed subjects or the same subject in the future. Alternatively, if the further psychological state of the subject shows that a determined adjustment did not entice an anxious subject to interact with a system, then the adjustment algorithm can be adjusted to determine a different adjustment for an anxious subject in future. In some embodiments, steps 230 and 240 may be added onto the end of method 100 of Fig. 1.

In other words, through a closed feedback loop the method can measure changes in the behaviour of a subject, such as a child, during and after interventions. Based on these reactions, the method can continuously learn what interventions work best for what type of anxiety/behaviour, and/or subject, and can store this in an effective anxiety-intervention mappings library or database. When a subject's reaction to an intervention does not have the desired effect, the method can try a second-best intervention to see if this works better. In this way, the method can continuously learn and adapt to create and use a library of the most effective anxiety-intervention mappings.

In some embodiments, based on the further psychological state, the same adjustment may be determined to be made in a clinical procedure room during the actual clinical procedure. In other words, if the determined adjustment was seen to be successful, i.e., affecting the subject's psychological state positively, then the same adjustment can be made during the real clinical procedure, e.g., playing the same music or providing the same displayed content. In some embodiments, based on the subject's psychological state and/or further psychological state, a planned duration of the clinical procedure may also be increased; the subject's caregiver may be invited to the clinical procedure, either in person or remotely through a display, e.g. activating tele-health communications; and/or health care professionals may be informed of the subject's psychological state during the procedure via content on a display, for example, a dashboard.

In other embodiments, the analysis of a subject's waiting room behaviour can be used longitudinally, e.g., to predict the subject's need for future support/guidance before and during future exams, procedures, and/or scans. The analysis of a subject's waiting room behaviour, i.e. determining a further psychological state, can also be used to evaluate and improve the effectiveness of subject experience-interventions and systems, or to stratify subjects towards the most optimal intervention for them. For example, if intervention A has a strong positive effect on most subjects, but intervention B does not, the adjustment algorithm can be automatically adapted to prioritize intervention B. If a specific intervention is more effective for subjects showing a certain behavioural pattern, this intervention can be prioritized for those specific subjects, etc.

For example, a database could be built that stores for each subject: 1) their starting psychological state, 2) which intervention/adjustment is determined and used, and 3) the effectiveness of that intervention/adjustment based the subject's subsequent psychological state. Optionally, the success rate of the actual clinical procedure can be used to update the database (i.e., how well was the subject prepared) and to interpret the interactions between points 1-3. Optionally, an exam card with information of the subject may be used to help interpret the interactions between points 1-3 (e.g., whether it is their first-time scan). This database can be used for analysis, e.g., to group certain subject profiles and assess effectiveness of interventions (for specific subject profiles or over time). Optionally, data about the subject's psychological state during and/or after a clinical procedure can be added to this database.

In some embodiments, further adjustments can be determined based on further psychological states. For example, if an adjustment (e.g., a dynamic floor projection) was seen to help the subject approach a preparatory system, but the subject is still anxious, further adjustments can be geared towards reducing anxiety, with higher levels of stimulations added once the subject is ready for it. In other words, a first adjustment could be to invite the subject to interact with the preparatory device via content displayed on displays in the environment. Determining that the subject is still anxious, even after approaching the preparatory device, a second adjustment could be made to play calming music. Determining that the subject is now calm, a third adjustment could be made for the preparatory device to flash its lights, inviting interaction.

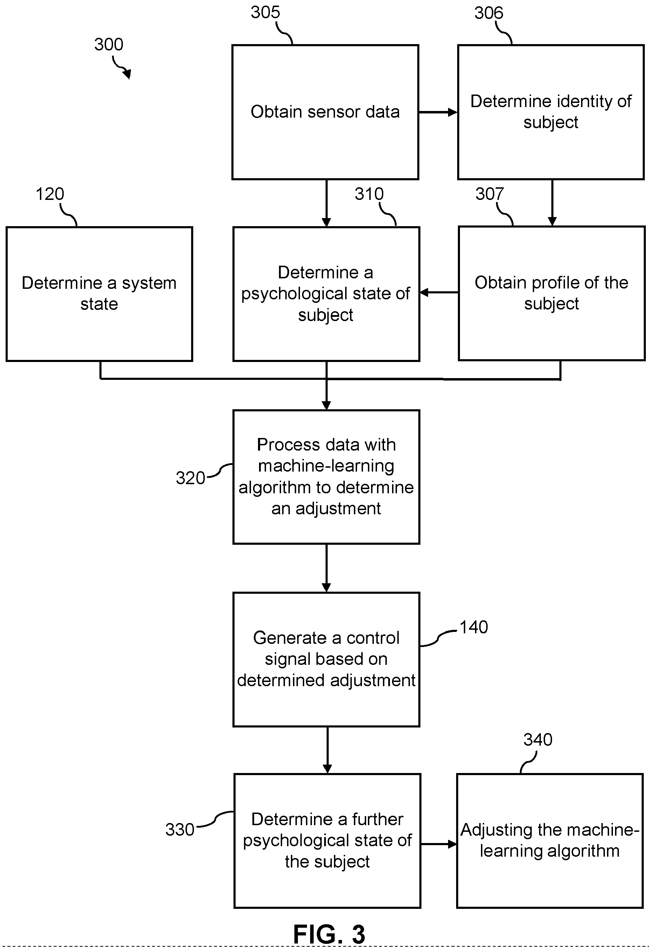

Referring now to Fig. 3, there is depicted a more in-depth flow diagram of a method 300 for controlling a clinical preparatory environment according to a proposed embodiment, wherein the clinical preparatory environment comprises a system configured to output a human-perceivable output

Steps 120 and 140 are substantially the same as have been described in relation to the method 100 in Fig. 1.

In step 305, sensor data is obtained describing at least one of a physiological and a psychological parameter of the subject. By obtaining sensor data, a physiological and/or a psychological parameter of the subject can be determined directly. For example, the sensor data may be obtained by cameras and/or microphones in the environment, and/or a heart rate monitor worn by the subject, and from these, the psychological state of the subject can be determined. Much like in step 205 of method 200, a physiological and/or a psychological parameter of the subject may comprise that the subject has been sitting still for twenty minutes or that the subject is fidgeting; that the subject has elevated heart rate; that the subject is clinging to their parents; or that the subject is simply looking away from the system. In some embodiments, visual analogue scales may also be used to determine a physiological and/or a psychological parameter of the subject. In some embodiments, semantic analysis of speech in the room may be used to determine a physiological and/or a psychological parameter of the subject. From this, a psychological state of the subject can then be determined, such as the subject being stressed or the subject not having their attention focused on the system. In some embodiments, the sensor data may be obtained by sensors embedded in the system itself and/or in wearables worn by subjects.

In embodiments in which sensor data is obtained by a ceiling camera, the data may describe at least one of: the position of the subject (i.e., location coordinates); facing-direction of the subject (i.e., nose vector or direction of sight); facial expression of the subject; heart rate of the subject; breathing rate of the subject; and the identity of the subject. Data from a ceiling camera may also be able to differentiate between children and parents. Technology of tracking people indoors is sufficiently covered in relevant scientific literature. Differentiation between individuals can be, for instance, based on colours of their clothes. Telling children from adults can also or alternatively be done by height sensors installed at the entrance or in the clinical preparatory environment itself. For example, a ceiling camera can be used to identify children that cling on to their parents and/or do not go to a preparatory system and/or show other anxiety related behaviour. Computer vision technology can be used to detect children, the system, and anxiety related behaviour via shapes, sizes, modelling, posture & behaviour pattern recognition and so on - all known in the field of security cameras. To enable this, information on the system location and/or characteristics, and/or an anxiety (behaviour) pattern library (database) may be used. Optionally, identification of the children can be realized through facial recognition, optical markers on cloths or wearables of the children, and/or via the use of a child identity profile library or database. In some embodiments, beam-forming microphones steered by a ceiling/wall camera may also be used to obtain sensor data.

In some embodiments, location coordinates of a system, such as a preparatory system within the environment may already be known, and so would not have to be determined via sensor data.

In some embodiments, the sensor data may comprise at least one of:
optical data; sound data; speech data; image data; movement data; accelerometer data; gyroscope data; haptic data; sweat data; electrodermal activity data; cardiovascular activity data; respiratory activity data; electroencephalography (EEG) data; temperature data; and touch data. All this data can be beneficial in determining the psychological state of a subject. Optical data can be acquired via a camera, for example. Sound or speech data can be acquired via a microphone, for example. Cardiovascular activity data can include different variability parameters such as SDNN, RMSSD, high/low frequencies, heart rate, and/or blood pressure, etc. Electrodermal activity data can include galvanic skin response and/or galvanic skin level.

In step 306, an identity of the subject is determined. In this embodiment, the identity of the subject is determined based on the obtained sensor data, such as, for example, camera data describing the subject's face. In other embodiments, however, the identity of the subject may be determined in any other suitable way, such as, for example, a user manually inputting the identity of the subject, or by obtaining an identity of the subject from an external database, such as for example, a hospital record.

In step 307, a profile of the subject is obtained based on the identity of the subject, wherein the profile describes at least one of: at least one trait of the subject; and at least one previous interaction of the subject with the system. In other words, the identity of the subject can be used to locate a profile of the subject from, for example, an internal or external database. The identity of the subject can be used to obtain a profile of the subject describing their traits, such as whether they are naturally anxious, their age, demographics, cultural and educational background, previous visits to the hospital and/or their previous interactions with a preparatory system. This information can then be used to more accurately determine the subject's psychological state and/or determine a more effective system adjustment. For example, if the subject's profile describes that they are naturally anxious, then this information is useful in determining their psychological state. Further, if the subject's profile describes that they previously interacted with the system only after a certain adjustment was made, then the same adjustment can be determined again or at least considered.

In step 310, a psychological state of the subject is determined based on the obtained sensor data and the obtained profile of the subject. For instance, optical data describing the facial expressions of the subject, speech data describing the subject's vocal volume, and the subject having a trait of being easily stressed can all be used to determine a stress value of the subject. In some embodiments, step 310 may comprise providing the obtained sensor data, and optionally the obtained profile of the subject, as input to a machine-learning algorithm trained to predict, for the sensor data (and, in some embodiments, also the profile of the subject), a psychological state of the subject.

In some embodiments, an anxiety knowledge data source, e.g., an anxiety pattern library, is used to interpret the sensor data and map it to certain anxiety levels, values, and/or profiles.

In step 320, the psychological state of the subject, the system state, and the profile of the subject are processed with a machine-learning algorithm trained to predict, based on the psychological state of the subject, the system state, and the profile of the subject, an adjustment for the system. Machine-learning algorithms can provide a more efficient and/or effective way to process information and provide outputs. For instance, the machine-learning algorithm may be a convolutional neural network or a random forest classifier, both beneficial for processing multiple inputs. Further, by considering the profile of the subject, a more beneficial and/or effective adjustment can be determined. For instance, if the subject's profile describes that they previously interacted with the system only after a certain adjustment was made, then the machine-learning algorithm can more favourably weight the same adjustment again in its decision making.

Further, the identity of the subject may be used to personalize adjustments. For example, a movie being provided on a display may be adjusted to call-out the subject's name. In another example, the movie may be adapted, e.g., to make it funnier for confident children vs warmer for stressed children.

In other embodiments, step 320 may involve processing only the psychological state of the subject and the system state with a machine-learning algorithm trained to predict, based on the psychological state of the subject and the system state, an adjustment for the system.

There are several types of machine-learning algorithms, such as, for example, convolutional neural networks (CNNs) and recurrent neural networks (RNNs). In embodiments of the present invention, the machine-learning algorithm may comprise CNN-based learning algorithms.

CNNs typically contain several layers, including a convolutional layer, a pooling layer, and a fully connected layer. The convolutional layer consists of a set of learnable filters and extracts features from the input. The pooling layer is a form of non-linear down-sampling, reducing the data size by combining the outputs of a plurality of neurons in one layer into a single neuron in the next layer. The fully connected layer connects each neuron in one layer to all the neurons in the next layer.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

In step 330, after generating a control signal in step 140, a further psychological state of the subject is determined. This may be determined by obtaining further sensor data after generating the control signal in a step (not shown) much like step 305. In step 340, the machine-learning algorithm can be adjusted based on the further psychological state of the subject.

In some embodiments, the adjustment may not be determined using a machine-learning algorithm but according to a simple fitness function, e.g., maximising the time a subject spends near a preparatory system. For example, the following simple adjustment algorithm could be employed:
With each image-frame obtained from a ceiling camera, extract location coordinates of both a child and their parent(s) and their `nose vectors'.
Compute Euclidian distance between child and preparatory system centre CS and distance between child and a parent CP (in case of two parents present, choose the one closest to the preparatory system at that moment). Keep track of cumulative sum of these variables in CSsum and CPsum respectively.

Every N seconds, check values of CSsum and CPsum and if CPsum/CSsum ratio is below a set threshold TH (for instance TH=1 meaning more time spend near the preparatory system) do nothing, otherwise trigger the preparatory system to prompt child to approach it by employing its actuators (sound, light etc).

Every M seconds check whether `nose' vector of a child is oriented towards the preparatory system, signifying child's interest in the toy, and if so employ actuators inviting child to engage in a play.

The above algorithm is one example of a simple adjustment algorithm; however, any other suitable adjustment algorithm may be used to determine an adjustment of the system.

In some embodiments, the subject may not be the patient preparing for a clinical procedure but instead their caregiver, parent, or sibling. Their psychological state may be assessed, and an adjustment determined for affecting their psychological state in exactly the same way as for a subject who is a patient.

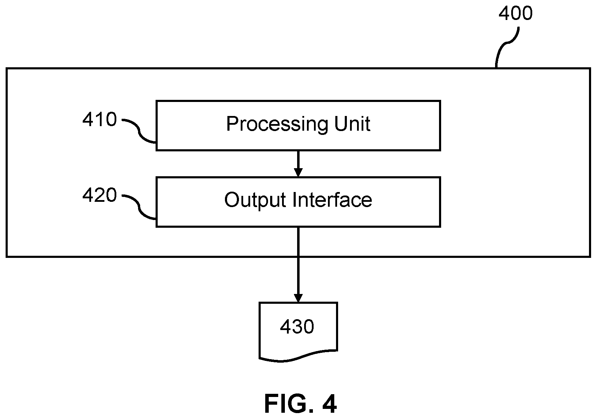

Referring now to Fig. 4, there is depicted a controlling apparatus 400 for controlling a clinical preparatory environment according to a proposed embodiment, wherein the clinical preparatory environment comprises a system configured to output a human-perceivable output. The controlling apparatus 400 comprises a processing unit 410 and an output interface 420.

The controlling apparatus 400 is configured to generate a control signal 430 for controlling the human-perceivable output of the system. The processing unit 420 is configured to determine a psychological state of a subject in the clinical preparatory environment; determine a system state describing an operating state of the system; and determine an adjustment for the system based on the psychological state of the subject and the system state. The output interface 420 is configured to generate, based on the determined adjustment, a control signal 430 for controlling the human-perceivable output of the system. In some embodiments, the controlling apparatus 400 may further comprise an input interface configured to obtain inputs, which may comprise subject information and/or sensor data.

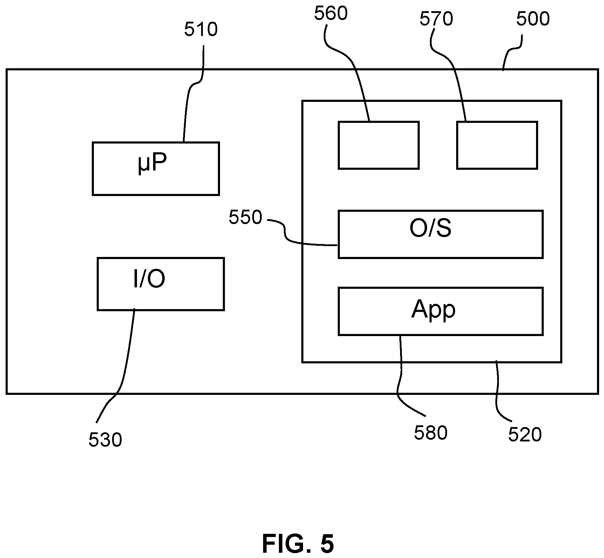

Fig. 5 illustrates an example of a computer 500 within which one or more parts of an embodiment may be employed. Various operations discussed above may utilize the capabilities of the computer 500. In this regard, it is to be understood that system functional blocks can run on a single computer or may be distributed over several computers and locations (e.g., connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520 and one or more I/O devices 530 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor-based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 560, source code 570, and one or more applications 580 in accordance with exemplary embodiments. As illustrated, the application 580 comprises numerous functional components for implementing the features and operations of the exemplary embodiments. The application 580 of the computer 500 may represent various applications, computational units, logic, functional units, processes, operations, virtual entities, and/or modules in accordance with exemplary embodiments, but the application 580 is not meant to be a limitation.

The operating system 550 controls the execution of other computer programs, and provides scheduling, input-output control, file and data management, memory management, and communication control and related services. It is contemplated by the inventors that the application 580 for implementing exemplary embodiments may be applicable on all commercially available operating systems.

Application 580 may be a source program, executable program (object code), script, or any other entity comprising a set of instructions to be performed. When a source program, then the program is usually translated via a compiler (such as the compiler 560), assembler, interpreter, or the like, which may or may not be included within the memory 520, so as to operate properly in connection with the O/S 550. Furthermore, the application 580 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 530 may include input devices such as, for example but not limited to, a mouse, keyboard, scanner, microphone, camera, etc. Furthermore, the I/O devices 530 may also include output devices, for example but not limited to a printer, display, etc. Finally, the I/O devices 530 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 530 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at start-up, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 580 and the O/S 550 are read, in whole or in part, by the processor 510, perhaps buffered within the processor 510, and then executed.

When the application 580 is implemented in software it should be noted that the application 580 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 550 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-3, and the controlling apparatus of Fig. 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method (100) for controlling a clinical preparatory environment, wherein the clinical preparatory environment comprises a system configured to output a human-perceivable output, the method comprising:
determining a psychological state of a subject (110) in the clinical preparatory environment;
determining a system state (120) describing an operating state of the system;
determining an adjustment for the system (130) based on the psychological state of the subject and the system state; and
generating, based on the determined adjustment, a control signal (140) for controlling the human-perceivable output of the system.

2. The method of claim 1, wherein the system is a preparatory system configured for preparing a subject for a clinical procedure via interaction with the subject.

3. The method of any of claims 1 or 2, wherein the human-perceivable output of the system comprises at least one of: lighting; sound; projections; content on a display; music; vocals; movement; touch; temperature; fragrance; speech; look; feel; and interaction style.

4. The method of any prior claim, wherein the system is an environmental system configured for controlling at least one environmental parameter of the preparatory environment.

5. The method of any prior claim, wherein determining a psychological state of a subject comprises: obtaining subject information (205) describing at least one of a physiological and a psychological parameter of the subject; and determining the psychological state of the subject (210) based on the obtained subject information.

6. The method of any of claim 5, wherein obtaining subject information comprises obtaining sensor data of the subject (305) describing at least one of a physiological and a psychological parameter of the subject.

7. The method of claim 6, wherein the sensor data comprises at least one of: optical data; sound data; speech data; image data; movement data; accelerometer data; gyroscope data; haptic data; sweat data; electrodermal activity data; cardiovascular activity data; respiratory activity data; electroencephalography (EEG) data; temperature data; and touch data.

8. The method of any prior claim, wherein a psychological state of the subject comprises at least one of: an attention direction of the subject; an anxiety value of the subject; a stress value of the subject; a verbal value of the subject; and a predominant emotion of the subject.

9. The method of any prior claim, wherein determining an adjustment for the system comprises processing the psychological state of the subject and the system state with an adjustment algorithm (220) to determine an adjustment for the system.

10. The method of claim 9, wherein the adjustment algorithm is a machine-learning algorithm (320) trained to predict, based on the psychological state of the subject and the system state, an adjustment for the system.

11. The method of any prior claim, wherein, after generating the control signal, the method further comprises:
determining a further psychological state of the subject (230); and
adjusting the adjustment algorithm (240) based on the further psychological state.

12. The method of any prior claim, wherein the method further comprises:
determining an identity of the subject (306);
obtaining a profile of the subject (307) based on the identity of the subject, wherein the profile describes at least one of: at least one trait of the subject; and at least one previous interaction of the subject with the system; and
at least one of:
determining the psychological state of the subject (310) based on the identity of the subject; and
determining an adjustment for the system (320) further based on the identity of the subject.

13. A computer program comprising code means for implementing the method of any preceding claim when said program is run on a processing system.

14. A controlling apparatus (400) for controlling a clinical preparatory environment, wherein the clinical preparatory environment comprises a system configured to output a human-perceivable output, the controlling apparatus comprising:
a processing unit (410) configured to:
determine a psychological state of a subject in the clinical preparatory environment;
determine a system state describing an operating state of the system; and
determine an adjustment for the system based on the psychological state of the subject and the system state; and
an output interface (420) configured to:
generate, based on the determined adjustment, a control signal (430) for controlling the human-perceivable output of the system.

15. The controlling apparatus of claim 14, wherein the system is a preparatory system configured for preparing a subject for a clinical procedure via interaction with the subject.
